Europäisches Patentamt

European Patent Office

Office européen des brevets

(11) Publication number: **0 556 489 A1**

## EUROPEAN PATENT APPLICATION

(21) Application number: **92200499.9**

(22) Date of filing: **19.02.92**

(51) Int. Cl.5: **C07C 5/333**, C07C 11/09, C07C 11/06, B01J 23/22

(43) Date of publication of application: **25.08.93 Bulletin 93/34**

(84) Designated Contracting States: **GB**

(71) Applicant: **SHELL INTERNATIONALE RESEARCH MAATSCHAPPIJ B.V. Carel van Bylandtlaan 30 NL-2596 HR Den Haag(NL)**

(72) Inventor: **Tromp, Petrus Joannes Josephus Badhuisweg 3 NL-1031 CM Amsterdam(NL)**
Inventor: **Groeneveld, Michiel Jan Badhuisweg 3 NL-1031 CM Amsterdam(NL)**
Inventor: **Arnoldy, Peter Badhuisweg 3 NL-1031 CM Amsterdam(NL)**
Inventor: **Clark, David Michael Badhuisweg 3 NL-1031 CM Amsterdam(NL)**

(54) **Process for the dehydrogenation of hydrocarbons.**

(57) A process for the dehydrogenation of hydrocarbons by contacting a feed containing one or more dehydrogenable hydrocarbons at an elevated temperature with a catalyst containing from 0.5 to 12 %w vanadia, calculated as vanadium metal relative to the weight of the catalyst, and from 0.01 to 1.0 gram equivalent/kg catalyst of at least one (earth-)alkali metal, on a refractory support, which process is carried out in the substantial absence of an oxidising gas.

This invention relates to a process for the dehydrogenation of hydrocarbons by contacting a feed containing one or more dehydrogenable hydrocarbons at an elevated temperature with a catalyst containing vanadia on a refractory support.

Processes of this kind are well known in the art for a long time, inter alia from US-A-2814650, US-A-3228992, US-A-4607129 and US-A-4644084. By these processes hydrocarbons can be dehydrogenated, e.g. to alkenes, with concomitant formation of hydrogen. There are several serious problems associated with these processes which are deleterious to the process economy. For example, coke may be formed on the surface of the catalyst so that the catalyst activity may become exhausted after a short period of time. The exhausted catalyst then needs to be replaced and/or to be regenerated. It is therefore an object of the present invention to provide a dehydrogenation process in which there is a reduced rate of coke formation.

It has now unexpectedly been found that a substantially reduced rate of coke formation can be accomplished by using a catalyst which contains, in addition to the vanadia, an alkali or an alkaline earth metal.

Accordingly, the present invention relates to a process for the dehydrogenation of hydrocarbons by contacting a feed containing one or more dehydrogenable hydrocarbons at an elevated temperature with a catalyst containing from 0.5 to 12 %w vanadia, calculated as vanadium metal relative to the weight of the catalyst, and from 0.01 to 1.0 gram equivalent/kg catalyst of at least one (earth-)alkali metal, on a refractory support, which process is carried out in the substantial absence of an oxidising gas.

According to the disclosures of US-A-3665049, which deals with propane dehydrogenation using a chromia containing catalyst, an enhanced catalyst life time can be accomplished by a special method of catalyst preparation. It is furthermore stated that traces of sodium oxide or potassium oxide can be incorporated into the chromia containing catalyst with the object of increasing the selectivity to propene. Hence, this document teaches away from improving the catalyst life time of a vanadia dehydrogenation catalyst by the addition of an alkali metal.

The process of the present invention is carried out in the substantial absence of an oxidising gas. In contrast herewith, oxidative dehydrogenation is carried out in the presence of an oxidising gas, such as molecular oxygen or halogen, with the object of removing the hydrogen produced in the dehydrogenation, e.g. by reaction with oxygen to form water (steam). US-A-4046833 discloses the use of supported vanadia catalysts in oxidative dehydrogenation of hydrocarbons which catalyst may contain an alkaline earth metal. This document teaches that by the particular choice of the catalyst system the coke formed on the catalyst is consumed by the oxygen present in the reaction mixture. Besides, it is known that in hydrocarbon conversion processes the presence of coke can be suppressed by operating in the presence of steam. Although this document provides a means for the enhancement of the catalyst life time in oxidative dehydrogenation, it does not teach a skilled person how to increase the catalyst life time in dehydrogenation processes which are carried out in the substantial absence of an oxidising gas.

The catalyst of the present process contains from 0.5 to 12 %w vanadia, calculated as vanadium metal relative to the weight of the catalyst. Preferably the catalyst contains from 1.5 to 9 %w and in particular from 3 to 6 %w vanadia, calculated as vanadium metal relative to the weight of the catalyst.

The catalyst contains, in addition to the vanadia, one or more (earth-)alkali metals. Preferred (earth-)alkali metals are the alkali metals, in particular lithium, potassium and cesium. The quantity of (earth-)alkali metal(s) present in the catalyst amounts to from 0.01 to 1.0 gram equivalent/kg catalyst, preferably from 0.05 to 0.5 gram equivalent/kg catalyst and in particular from 0.1 to 0.3 gram equivalent/kg catalyst.

It has also been found that a further reduction in the rate of coke formation can be accomplished by using a catalyst which contains, in addition to vanadia and the (earth-)alkali metal, a relatively small quantity of a noble metal, such as platinum or palladium. It appeared, surprisingly, that when the (earth-)alkali metal is not incorporated into the catalyst the presence of a noble metal does not decrease appreciably the rate of coke formation. It has furthermore been found that by incorporating an element selected from Group 3a of the Periodic Table into the catalyst which contains vanadia, the (earth-)alkaline metal and the noble metal a further decrease in the rate of coke formation can be accomplished. A metal selected from the group consisting of germanium, tin or lead can be used instead of the element selected from Group 3a of the Periodic Table.

Accordingly, the catalyst contains preferably one or more noble metals, in addition to vanadia and the (earth-)alkali metal. The noble metals are those metals of Group VIII of the Periodic Table of Elements which have an atomic number of at least 44, for example rhodium and osmium. Preferred noble metals are palladium and in particular platinum. The noble metal(s) is/are present in a quantity of from 0.00005 to 0.01 gram atom/kg catalyst, preferably from 0.0002 to 0.008 gram atom/kg catalyst and in particular from 0.0005 to 0.005 gram atom/kg catalyst.

The catalyst contains more preferably, in addition to the vanadia, the (earth-)alkali metal and the noble metal, an element selected from Group 3b of the Periodic Table and/or a metal selected from the group consisting of germanium, tin or lead. Most preferably a Group 3b element is present, in particular one or more of the lanthanides, more in particular cerium. Group 3b elements may be present in a quantity of from 0.01 to 1 gram atom/kg catalyst, typically from 0.03 to 0.8 gram atom/kg catalyst, more typically from 0.1 to 0.5 gram atom/kg catalyst. The metal(s) selected from germanium, tin and lead may be present in a quantity of from 0.01 to 0.5 gram atom/kg catalyst.

The refractrory support may be of any available type, such as supports comprising magnesium oxide, titanium oxide, magnesium aluminate or aluminium phosphate. Preferred refractory supports comprise silica, whereas alumina containing supports are the most preferred refractory supports.

The alumina containing supports are suitably obtained from porous alumina which can be any of the variety of available aluminas, such as alumina hydrates, alumina gel and activated alumina. Very suitable aluminas are found to be those which have a surface area ranging from 10 $m^2/g$ to 500 $m^2/g$, preferably from 50 $m^2/g$ to 400 $m^2/g$. In a preferred embodiment the alumina containing support is gamma-alumina.

Excellent results can be obtained with catalysts which contain from 3.5 to 5 %w of vanadia, calculated as vanadium metal relative to the weight of the catalyst, from 0.13 to 0.26 gram equivalent/kg catalyst of an alkali metal and from 0.001 to 0.0025 gram atom/kg catalyst of platinum or palladium on a gamma-alumina support.

The catalyst can be prepared by methods known in the art, for example by impregnating or otherwise providing the refractory support with the desired quantity of vanadia, the (earth-)alkali metal(s), the optional noble metal(s), the optional Group 3a element(s) and the optional metal(s) selected from germanium, tin and lead. After the impregnation, the refractory support is suitably dried and calcined. Calcining is preferably carried out by heating in an oxygen containing atmosphere at a temperature in the range of from 300 to 800 $°C$.

The catalyst may be brought into any suitable shape prior to the impregnation, or after the impregnation. Various techniques for shaping the catalyst are available in the art, such as extrusion and compression moulding.

Before using the catalyst in the process of the invention, the catalyst may be subjected to heat, preferably at the same or approximately the same temperature as the temperature employed in the dehydrogenation process, in the presence of an inert gas, such as argon or nitrogen, or in the presence of a reactive gas, such as hydrogen or ammonia. A freshly prepared catalyst may be subjected to such a heat treatment, as well as a catalyst which is obtained from a regeneration step, as described hereinafter.

In the process of the invention the catalyst is contacted with a feed containing one or more dehydrogenable hydrocarbons. A skilled person will appreciate that dehydrogenable hydrocarbons contain at least two carbon atoms to each of which one or more hydrogen atoms are bound and, upon loss of hydrogen, at least two of these carbon atoms become part of a cyclic structure and/or an unsaturated moiety. The unsaturated moiety can be olefinic or aromatic. The feed typically contains one or more linear or branched alkanes, in particular $C_2$ - $C_{10}$ alkanes, more in particular $C_2$ - $C_5$ alkanes. A very suitable feed contains alkanes which have been prepared in a Fischer-Tropsch synthesis. Preferably the feed contains propane and/or isobutane.

The present process is suitably carried out in a continuous mode of operation, preferably using a fluidized bed or a moving packed catalyst bed. The bed of catalyst may move upwards or downwards.

In the present process the feed is contacted with the catalyst for a suitable period of time in order to accomplish the desired level of conversion of the hydrocarbon(s) in the feed. More suitably the feed is contacted with the catalyst during at least 0.001 seconds, preferably during from 0.01 to 5 seconds and in particular during from 0.05 to 2 seconds. To a skilled person it will immediately be clear that the residence time required for a certain conversion level may depend, amongst other things, on the density of the catalyst particles in a reaction zone. When using a packed catalyst bed, having in general a higher density than a fluidized catalyst, a lower residence time may be employed than when using the fluidized catalyst.

The temperature at which the feed is contacted with the catalyst is suitably 400 $°C$ or higher, preferably higher than 500 $°C$. The temperature is suitably kept below 800 $°C$, in particular below 750 $°C$, in order to' further limit the formation of coke. Very good results can be accomplished at a temperature in the range of from 550 to 700 $°C$.

The pressure in the present process can be varied within wide ranges. It is preferred that the pressure is such that at the prevailing temperature the feedstock is substantially in its gaseous phase. The pressure is preferably in the range of from 0.1 to 5 bar. This can be advantageous since no expensive compressors are necessary. Other gaseous materials may be present during the conversion, such as steam and/or nitrogen.

As stated hereinbefore, in oxidative dehydrogenation processes, known in the art, the feed comprises an oxidising gas, in addition to the hydrocarbon(s). In such processes the hydrogen, which is formed as a result of the dehydrogenation, will be converted. As hydrogen is a valuable material, the process of the invention is intended to be carried substantially in the absence of an oxidising gas, preferably in the complete absence thereof.

Although in the present process the rate of coke formation may be lower than in comparable prior art processes, the catalyst may still loose activity as a result of coke formation. It could be advantageous to regenerate the catalyst in a regeneration step, preferably, by subjecting the catalyst after having been contacted with the feedstock to a treatment with an oxidizing gas, such as air. A continuous regeneration, similar to the regeneration carried out in a fluidized catalytic cracking reaction, is specially preferred.

If coke formation does not occur at a rate which is too high it would be possible to arrange for a process in which the residence time of the catalyst particles in the reaction zone is longer than the residence time of the feedstock in the reaction zone. Suitably the residence time of the catalyst is from 1 to 50 times the residence time of the feedstock.

The dehydrogenation products obtained in the present process may be recovered and purified by any suitable technique, such as distillation. Unconverted starting hydrocarbon(s) may be isolated as well and, if desired, fed to a subsequent dehydrogenation, optionally, after combining with fresh feed.

The present invention will be further illustrated by means of the following examples.

EXAMPLES 1 - 11

Catalyst preparation

Samples of a gamma-alumina support (crushed extrudates, surface area 190 $m^2/g$, pore volume 0.85 ml/g, pre-calcined at 700 °C during 16 hours) were impregnated with aqueous solutions of vanadium oxalate and optionally one or more compounds selected from lithium hydroxide, potassium nitrate, cesium hydroxide, platinum chloric acid, palladium nitrate and cerium nitrate by mixing a sample of the support with a quantity of the aqueous solution which is sufficient to fill the pores of the support, the quantity of vanadium, alkali metal, platinum, palladium and cerium in the solution being the quantity to be supplied to the support. Subsequently the mixtures were dried and calcined.

For example, a catalyst containing about 5.0 %w vanadia, calculated as vanadium metal relative to the weight of the catalyst, 0.13 gram equivalent of potassium/kg catalyst and 0.001 gram atom of platinum/kg catalyst was prepared as follows. The following aqueous solutions were combined: 18.43 g of a solution of vanadium oxalate (vanadium content 6 %w), 1.11 g of a solution of potassium nitrate (potassium content 10 %w) and 1.03 g of a solution of platinum chloric acid (platinum content 0.43 %w). Water was removed from the combined solutions by evaporation until the volume of the solution was 17 ml. The solution thus obtained was added portion wise to 20 g of the pre-calcined support. During the addition the support was kept in motion to ensure that all support particles were wetted. About 90 minutes after the last portion of solution was added, the support was dried by heating in an oven in air at 120 °C for 4 hours and subsequently calcined by heating at 450 °C in air for 1 hour.

For each of the catalysts prepared the contents of vanadium, alkali metal, platinum, palladium and cerium are given in Table 1.

Dehydrogenation of isobutane

A quartz tubular reactor (2mm diameter) was charged with a 200 mg sample of one of the catalysts obtained. The catalyst bed thus formed was heated at 650 °C in a flow of 0.6 Nl argon/h. At certain intervals of time the flow of argon was interrupted by feeding a pulse of isobutane (2.4 Nml) at the same flow rate as the argon flow. From the composition of the gases leaving the reactor, as measured by gas-liquid chromatography (GLC), the conversion of isobutane was calculated. The main volatile byproducts were methane, ethane, ethene, propane, propene, n-butane and n-butenes. The rate of coke formation was estimated from the carbon mass balance calculated from the GLC analysis. The residence time of the gas in contact with the catalyst was estimated, taking into account the temperature and the average pressure (about 1.2 bar) of the reaction mixture in the reactor. The residence time was found to be approximately 0.3-0.4 s. The results of the fifth pulse in each of the Examples 1-11 are given in Table 1.

TABLE 1

| Example | Catalyst, content [1] of | | | | | | Isobutane conversion | Rate of coke formation [2] |
| | V | Cs | K | Pt | Pd | Ce | | |
| | % w | g atom/kg | | mg atom/kg | | g atom/kg | % mole | |
| 1 [3] | 3.5 | - | - | - | - | - | 77 | 1.0 |
| 2 | 3.5 | 0.13 | - | - | - | - | 76 | 0.5 |
| 3 [3] | 3.5 | - | - | 1.0 | - | - | 80 | 1.1 |
| 4 [3] | 3.5 | - | - | 1.0 | - | 0.36 | 77 | 0.9 |
| 5 | 3.5 | 0.13 | - | 1.0 | - | - | 71 | 0.3 |
| 6 | 3.5 | 0.13 | - | 1.0 | - | 0.36 | 57 | 0.2 |
| 7 | 3.5 | 0.13 | - | 2.6 | - | - | 71 | 0.5 |
| 8 [3] | 5.0 | - | - | - | - | - | 79 | 1.0 |
| 9 | 5.0 | - | 0.13 | - | - | - | 70 | 0.4 |
| 10 | 5.0 | - | 0.13 | 1.0 | - | - | 74 | 0.2 |
| 11 | 5.0 | - | 0.13 | - | 0.94 | - | 79 | 0.2 |

[1]  Relative to the weight of the catalyst, vanadium content calculated as vanadium metal

[2]  Arbitrary units; results of Examples 1 and 8 are 1.0 by definition; results of Examples 2 - 7 to be compared with the result of Example 1 and results of Examples 9 - 11 to be compared with the result of Example 8

[3]  For comparison, not according to the invention

EXAMPLES 12-16

The catalyst preparation and the dehydrogenation according to the methods applied in Examples 1-11 were substantially repeated but with the difference that propane was used instead of isobutane and that the temperature in the dehydrogenation step was 700 °C instead of 650 °C. The main volatile byproducts were methane, ethane and ethene. The results are given Table 2.

# EP 0 556 489 A1

TABLE 2

| Example | Catalyst, content [1]) of | | | | Propane conversion % mole | Rate of coke formation [2]) |
|---|---|---|---|---|---|---|
| | V | Li | K | Cs | | |
| | % w | g atom/kg | | | | |
| 12 [3]) | 3.5 | - | - | - | 81 | 1.0 |
| 13 | 3.5 | 0.13 | - | - | 77 | 0.8 |
| 14 | 3.5 | - | 0.13 | - | 72 | 0.6 |
| 15 | 3.5 | - | 0.26 | - | 56 | 0.3 |
| 16 | 3.5 | - | - | 0.13 | 69 | 0.4 |

[1]) Relative to the weight of the catalyst, vanadium content calculated as vanadium metal
[2]) Arbitrary units; result of Examples 12 is 1.0 by definition; results of Examples 13 - 16 to be compared with the result of Example 12
[3]) For comparison, not according to the invention

**Claims**

1. A process for the dehydrogenation of hydrocarbons by contacting a feed containing one or more dehydrogenable hydrocarbons at an elevated temperature with a catalyst containing from 0.5 to 12 %w vanadia, calculated as vanadium metal relative to the weight of the catalyst, and from 0.01 to 1.0 gram equivalent/kg catalyst of at least one (earth-)alkali metal, on a refractory support, which process is carried out in the substantial absence of an oxidising gas.

2. A process as claimed in claim 1, characterised in that the catalyst contains from 3 to 6 %w vanadia, calculated as vanadium metal relative to the weight of the catalyst.

3. A process as claimed in claims 1 or 2, characterised in that the (earth-)alkali metal(s) are selected from the alkali metals.

4. A process as claimed in any of claims 1 to 3, characterised in that the quantity of (earth-)alkali metal present in the catalyst amounts to from 0.05 to 0.5 gram equivalent/kg catalyst.

5. A process as claimed in claim 4, characterised in that the quantity of (earth-)alkali metal present in the catalyst amounts to from 0.1 to 0.3 gram equivalent/kg catalyst.

6. A process as claimed in any of claims 1 to 5, characterised in that the catalyst contains one or more noble metals in a quantity of from 0.00005 to 0.01 gram atom/kg catalyst.

7. A process as claimed in claim 6, characterised in that the noble metal(s) is/are palladium and/or platinum.

8. A process as claimed in claims 6 or 7, characterised in that the noble metal(s) is/are present in a quantity of from 0.0005 to 0.005 gram atom/kg catalyst.

9. A process as claimed in any of claims 6 to 8, characterised in that the catalyst contains an element selected from Group 3b of the Periodic Table in a quantity of from 0.01 to 1 gram atom/kg catalyst.

10. A process as claimed in claim 9, characterised in that the element selected from Group 3b of the Periodic Table is cerium.

11. A process as claimed in claims 9 or 10, characterised in that the the element selected from Group 3b of the Periodic Table is present in a quantity of from 0.1 to 0.5 gram atom/kg catalyst.

6

12. A process as claimed in any of claims 1 to 11, characterised in that the refractory support is an alumina containing support.

13. A process as claimed in claim 12, characterised in that the alumina containing support is gamma-alumina.

14. A process as claimed in any of claims 1 to 13, characterised in that the catalyst contains from 3.5 to 5 %w of vanadia, calculated as vanadium metal relative to the weight of the catalyst, from 0.13 to 0.26 gram equivalent/kg catalyst of an alkali metal and from 0.001 to 0.0025 gram atom/kg catalyst of platinum or palladium on a gamma-alumina support.

15. A process as claimed in any of claims 1 to 14, characterised in that the feed contains alkanes which have been prepared in a Fischer-Tropsch synthesis.

16. A process as claimed in any of claims 1 to 14, characterised in that the feed contains propane and/or isobutane.

17. A process as claimed in any of claims 1 to 16, characterised in that the feed is contacted with the catalyst at a temperature of from 500 to 750 °C.

18. A process as claimed in claim 17, characterised in that the feed is contacted with the catalyst at a temperature of from 550 to 700 °C.

19. A process as claimed in any of claims 1 to 18, characterised in that the feed is contacted with the catalyst during from 0.01 to 5 seconds.

20. A process as claimed in claim 19, characterised in that the feed is contacted with the catalyst during from 0.05 to 2 seconds.

21. A process as claimed in claim 1 and substantially as hereinbefore described with particular reference to Examples 2, 5 - 7, 9 - 11 and 13 - 16.

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5 ) |
|---|---|---|---|
| X | JOURNAL OF APPLIED CHEMISTRY OF USSR. vol. 62, no. 8, August 1989, NEW YORK US pages 1806 - 1809; O. V. ZOLOTAREV ET AL.: 'Effect of NiO and Na2O on the acid-base and catalytic properties of V2O5/gamma-Al2O3 catalysts of dehydrogenation of ethylbenzene' * the whole article * | 1-6 | C07C5/333 C07C11/09 C07C11/06 B01J23/22 |
| A | CHEMICAL ABSTRACTS, vol. 114, no. 21, 27 May 1991, Columbus, Ohio, US; abstract no. 206342U, A.A. SAID ET AL.: 'Dehydration-dehydrogenation of isopropyl alcohol on pure divanadium pentoxide and doped with alkali metal oxides' page 743 ; column 2 ; & J. Indian Chem. Soc. 1990, 67(9), 734-9 * abstract * | 1 | |
| A | EP-A-0 403 462 (FINA RESEARCH) * page 3, line 2 - page 3, line 4 * * examples 1,2 * | 1 | TECHNICAL FIELDS SEARCHED (Int. Cl.5 ) |
| A | DE-B-2 741 625 (LUMMUS CO.) | 1 | C07C B01J |
| D,A | US-A-4 607 129 (F.M. LEE) * column 2, line 40 * | 1 | |

The present search report has been drawn up for all claims

| Place of search | Date of completion of the search | Examiner |
|---|---|---|
| BERLIN | 01 OCTOBER 1992 | PROBERT C. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document